# EUROPEAN PATENT APPLICATION

(11) **EP 0 967 201 A1**
(43) Date of publication of application: **29.12.1999**
(21) Application number: 98109154.9
(22) Date of filing: 20.05.1998
(51) Int. Cl.: C07C 311/02, C07C 311/10, C07C 311/15, A61K 31/18

(54) **Pharmaceutical agents containing sulfonamids as matrix metalloproteinase inhibitors**

(71) Applicant: Roche Diagnostics GmbH, 68298 Mannheim (DE)
(72) Inventor: Grams, Frank, Dr.Dipl.-Chem., 68219 Mannheim (DE); Zimmermann, Gerd, Dr.Dipl.-Chem., 68309 Mannheim (DE); Krell, Hans-Willi, Dr.Dipl.-Biol., 82377 Penzberg (DE); Kucznierz, Ralf, Dr.Dipl.-Chem., 67098 Bad Dürkheim (DE)

(57) **Abstract**

The invention concerns the use of sulfonamids of formula I as matrix metalloprotease (MMP) inhibitors 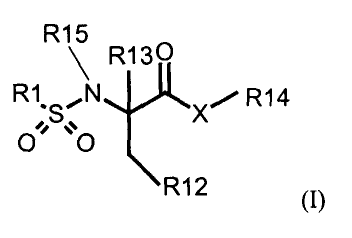 wherein R1, X, R12, R13, R14 and R15 have the above stated meanings as well as their racemates, diastereomers, pharmacologically acceptable salts or prodrugs thereof, to produce pharmaceutical agents for the treatment of diseases where MMP activity is involved.

## Description

The invention concerns the use of sulfonamids as matrix metalloprotease (MMP) inhibitors.

In normal tissue there is an equilibrium between synthesis and degradation. Extracellular matrix is degraded by proteases which belong to the family of matrix metalloproteases. Among them are the collagenases, gelatinases and stromelysins. Normally there are endogenic inhibitors for these catabolic enzymes such as α₂ macroglobulines and TIMP (= tissue inhibitor of metalloproteases (MMP)) so that an excessive degradation of extracellular matrix does not occur.

At least 15 different and yet highly homologous MMP species have been characterized, including the interstitial fibroblast collagenase (MMP-1, HFC), the neutrophil collagenase (MMP-8, HNC), two gelatinases (gelatinase A or MMP-2 and gelatinase B or MMP-9), stromelysins (such as HSL-1, MMP-3) and Matrilysin (MMP-7). These proteinases share a number of structural and functional features but differ somewhat in their substrate specificity. Only the interstitial collagenases are capable of cleaving type I, II and III native triple-helical collagens at a single bond with the production of fragments 3/4 and 1/4 of the native chain length. This lowers the collagen melting point and makes them accessible to further attack by other matrix degrading enzymes.

However, the uncontrolled excessive degradation of this matrix is a characteristic of many pathological states such as e.g. in the clinical picture of angiogenesis, tumor progression, sun-induced skin-aging, emphysema, cardiovascular diseases like restinosis, arteriosclerosis and platelet aggregation, rheumatoid arthritis, osteoarthritis, periodontal diseases, multiple sclerosis, in the formation of tumour metastases, corneal ulceration, inflamative diseases and diseases, where the presence of bacteria results in the release of MMPs, like bacterial Meningitis.

It can be assumed that the pathogenesis of these clinical pictures can be favourably influenced by the administration of matrix metalloprotease inhibitors. A number of compounds are known in the literature (see e.g. the review article of Beckett RP, Davidson AH, Drummond AH, Huxley P, Whittaker M. Drug Disc. T. (1996)1: 16-26.)) or are described in the patent literature, these mainly being peptides with a hydroxamic acid residue, a thiol or phosphinic group as a zinc binding group (see e.g. WO-A-9209563 by Glycomed, EP-A-497 192 by Hoffmann-LaRoche, WO-A-9005719 by British Biotechnology, EP-A-489 577 by Celltech, EP-A-320 118 by Beecham, US-A-459 5700 by Searle among others). WO97/44315 (Warner-Lambert Company) discloses biphenylsulfonamide matrix metalloproteinase inhibitors, but the described compounds are excluded from the present invention.

Some of the disclosed compounds have a high activity as inhibitors of matrix metalloproteases but only have a very low oral availability.

It has now been found that the claimed sulfonamids are very efficacious as matrix metalloprotease inhibitors and are orally available.

The present invention therefore concerns the use of substances of the general formula I

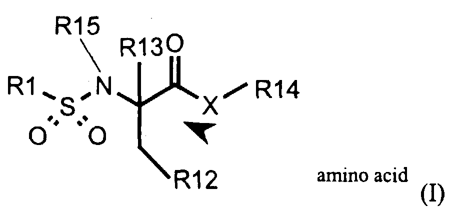

If the residue R1 shows certain hydrophobicity values, improved inhibition of MMPs is observed. It is advantageous, if the first three backbone atoms in R1 adjacent to the sulfonamide structural element in the inhibitors of die invention consists of carbon atoms. A useful hydrophobicity parameter is the logP which can be calculated using "PCModels CLOGP3" provided by Daylight Chemical Information Systems, Inc (1993). The coefficients are based on Hansch, C. & Leo, A.: Substituent Constants for Correlation Analysis in Chemistry and Biology. Wiley Interscience New York (1979), the algorithm is based on Chou, J. & Jurs, P., J. Chem. Inf. Comput. Sci. 19, 172 (1979). The fragments are calculated as molecules H-R1 and not as radicals or ions.

### Examples:

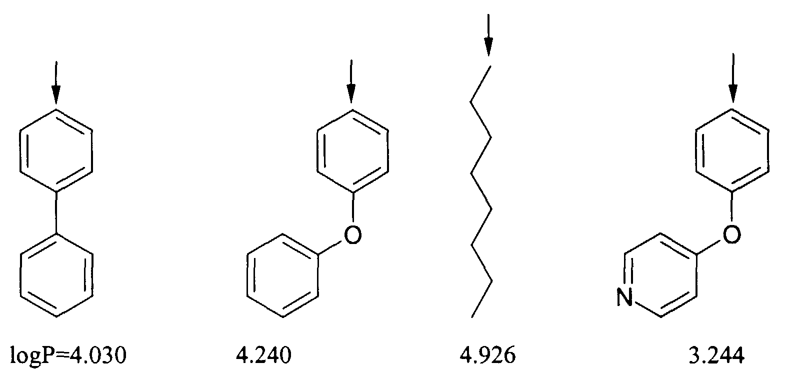

(The first atom in the chain is marked by an arrow)
The logP-values of R1 in the compounds of the invention as calculated for H-R1 show values between 1.5 to 5.5, preferred 2 to 5, more preferred between 2.5 and 5
It is also advantageous if there is no high local hydrophilicity along the chain, therefore heteroatoms along the chain should be always spaced by at least 2 carbon atoms.

The length of the relaxed residue R1 is in the range from 6 to 10 Ångstroms, preferred between 7 and 9 Ångstroms. The maximal width of R1 is 3.5 Å. All dimensions are calculated without H-atoms. The volume of the connolly surface of R1 is between 150 and 250 Å³, preferred between 170 and 230 Å³ (calculated with Insight II (MSI) San Diego).

Therefore new MMP inhibitors are compounds which are represented by the general formula I, 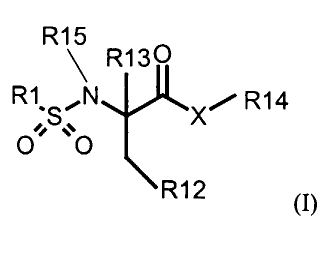 wherein
R₁ shows a logP-value between 1.5 and 5.5 if calculated as H-R₁ with the proviso that at least the beginning three backbone atoms of the chain are carbon atoms, with the proviso that there are maximal 3 heteroatoms in the chain and with the proviso that there are at least 2 carbon atoms between each pair of heteroatoms in the chain.

Alternatively R₁ can be defined as representing a chain which is optionally substituted once to three times and the chain consists of 7 to 11 backbone atoms comprising maximal 3 heteroatoms and the proviso that at least the beginning three backbone atoms of the chain are carbon atoms.

More preferable R₁ is one of the following residues: 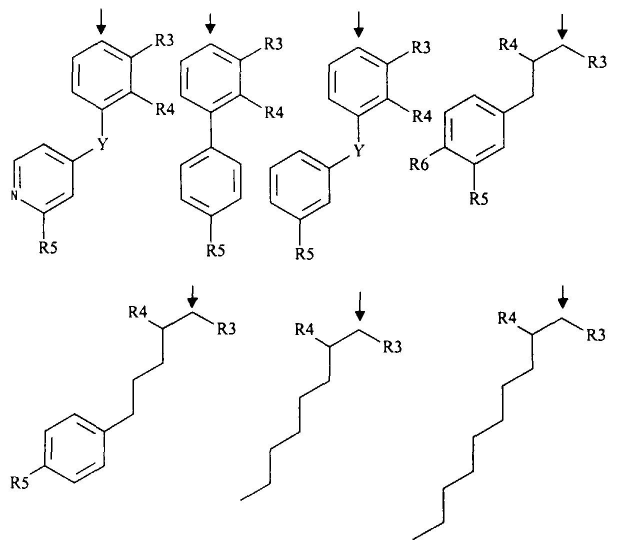 whereby
R3 and R4 independently have the one of the meanings H, C₁-C₁₀-alkyl, aryl, aryl-C₁-C₆-alkyl, C₁-C₁₀-heteroalkyl, heteroaryl, heteroaryl-C₁-C₆-alkyl, heteroaryl-C₁-C₆-heteroalkyl, aryl-C₁-C₆-heteroalkyl, and
R3 and R4 are optionally in the case of a phenyl substitution in addition independently of each other NR7R8
   R7 and R8 is independently of each other hydrogen or lower alkyl
R3 and R4 are optionally in the case of an alkyl substitution in addition independently of each other COR10
   R10 is hydroxy or C1-C10 heteroalkyl
R5 and R6 independently have the meanings H, OH, halogen, lower alkyl, lower alkenyl, or lower heteroalkyl, preferable at least one is hydrogen,
Y has the meanings O, S, NH, (CH₂)ₙ, n is 1, 2 or 3,

Preferable at least one of R3 and R4 is hydrogen, more preferred R4 is hydrogen.

Preferable at least one of R5 and R6 is hydrogen, more preferred R6 is hydrogen.
R5 and R6 independently have the preferred meanings H, OH, Halogen, lower alkyl, or lower heteroalkyl,
Y is preferably O,

Highly preferred are the following residues for R₁: 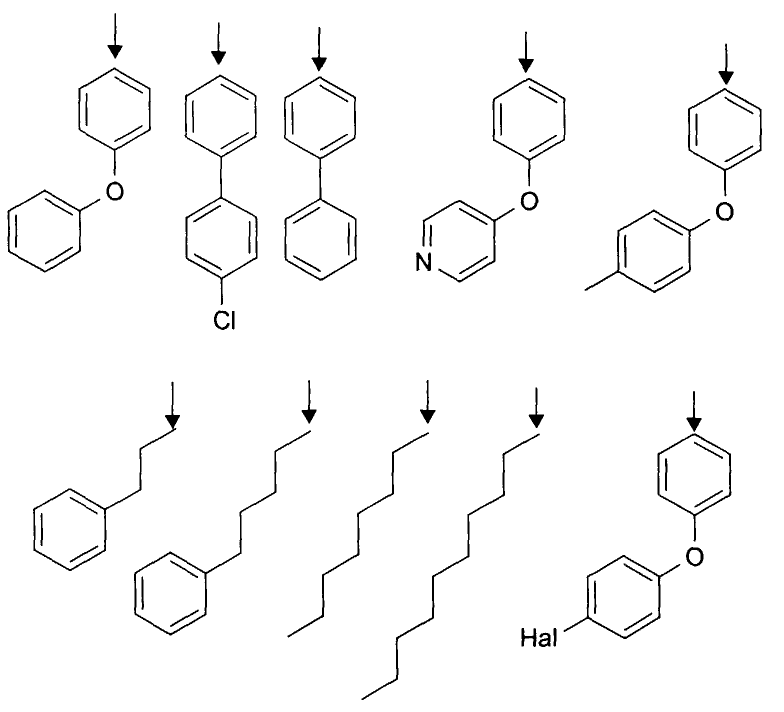
R12 is a residue of 5 to 21 backbone atoms, said residue being saturated or unsaturated, linear or branched, and contains optionally homocyclic or heterocyclic structures,
R12 is preferably aralkyl or represented by -(CH2)n-W-R12' with n = 0 - 4 and W = S, O, NH and R12' is the remaining moiety of R12, but not H or methyl.
R13 represents H, lower alkyl, lower alkenyl, phenyl or benzyl.
R13 is preferably H
R14 stands for H, alkyl, alkenyl, aryl or aralkyl. Alternately, it can form together with X, if X is N, a C-terminal protected or unprotected aminoacid. Protection means an ester of lower alkyl, alkenyl, aryl or aralkyl.
R14 may also be represented by the formula -CH(R15')-O-CO-R16 wherein R15' stands for H, alkyl, alkenyl, aralkyl or aryl and R16 has the meaning of optionally substituted alkyl, alkoxy, aralkyl, aralkyloxy. In addition, R15' and R16 may form a five or sixmembered ring, which may be optionally substituted or condensed with further carbocyclic or heterocyclic rings. Other examples of prodrugs are well known and can be found for example in Drugs of the Future 16, 443 (1991),
with the proviso, that R14 is not H, if X stands for O and the configuration of the C-atom with the substituents R13, N, CO and CH₂R12 is derived from a D-aminoacid with R13 is H; and with the provisio that R14 cannot be H, C₁-C₆-alkyl, benzyl or tert. butyl if X is O and R1 is a biphenyl residue; and with the provisio that R14 can be H if X denotes O, R1 is biphenyl and if R12 is represented by the group -(CH₂)n-W-R12' wherein W denotes S and R12' is the remaining moiety of R12, but not H or methyl,
R15 is H, lower alkyl, aryl, heteroaryl, heteroaryl-lower-alkyl, Preferred is H.
X stands for O or N. Preferably X stands for O.

We have found that it is favorable not to have a lone standing hydrogen bond acceptor in the chain of R1 (e.g. CO or CS), therefore these compounds are not claimed. Due to metabolic instability we are also not claiming compounds which comprise amid or ester groups in the chain of R1.

The compounds of the invention also comprise racemates, diastereomers pharmacologically acceptable salts and prodrugs of compounds of general formula I. Examples for prodrugs are esters like ethyl or 2,2-dimethylpropionyloxymethyl or ethoxycarbonyloxymethyl or 3-oxo-1,3-dihydro-isobenzofurane-1-yl esters which are metabolized in vivo to the compounds of formula I.

The following compounds of formula I are new,
wherein R₁ is represented by the following residues: 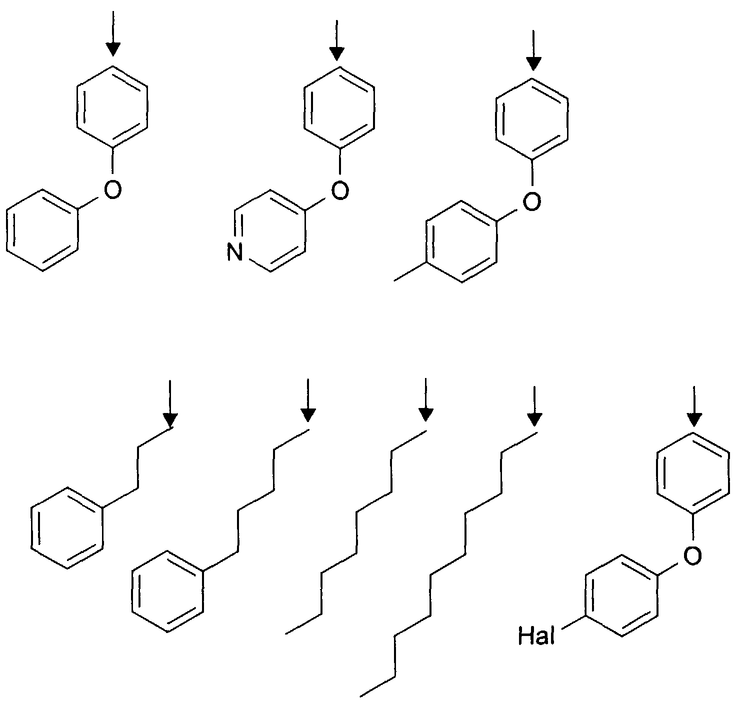
R15, R13, X and R14 have the above stated meanings,
and R12 is a residue of 5 to 21 backbone atoms, said residue being saturated or unsaturated, linear or branched, and contains optionally homocyclic or heterocyclic structures, represented by -(CH2)n-W-R12' with n = 0 - 4 and W = S, and R12' is the remaining moiety of R12, but is not H or methyl.

Backbone atoms are C, N, O and S, hetero atoms denote N, O and S.

Peptide group denotes -C(O)-NH- which is bound to two adjacent C atoms.

Chain denotes in R1 for the longest linear backbone atom chain wherein aromatic rings if part of the chain count as maximally 4 backbone atoms. Aromatic rings per se as part of the chain do not represent substitutes. For examples the chain length of -(CH₂)₃-CH(CH₃)-CH₂-CH₃ equals 6 (1 Subst.), -(CH₂)₃-Ph(p-CH₂-CH₃) equals 9, -(CH₂)₃-Ph(m-CH₂-CH₃) equals 8 and -(CH₂)₃-Ph equals 7 (all 0 Subst.).

Alkali salts, ammonium salts, trifluoroacetates, hydrochlorides or other salts of mineral acids are used above all as pharmacologically acceptable salts which are usually produced for example by titrating the compounds with inorganic or organic bases or acids such as e.g. sodium or potassium bicarbonate, sodium hydroxide solution, potassium hydroxide solution, aqueous ammonia or amines such as trimethylamine or triethylamine, trifluoroacetic acid or hydrochloric acid. The salts are usually purified by precipitation from water/acetone.

In all cases lower alkyl should represent a straight-chained or branched C₁-C₄ alkyl group such as e.g. methyl, ethyl, propyl, isopropyl, butyl or isobutyl, in particular methyl, ethyl, propyl, and isobutyl.

Alkyl should represent a straight-chained or branched C1-C10 alkyl group such as eg. propyl, isopropyl, butyl or isobutyl, in particular methyl, ethyl, propyl, isobutyl, pentyl, hexyl, heptyl, octyl, nonyl, decyl.

Lower alkenyl denotes unsaturated residues with 2,3 or 4 carbon atoms such as allyl, or but-2-enyl, but above all allyl.

Lower heteroalkyl denotes for an alkyl residue where 1, 2 or 3 C-atoms are substituted by heteroatoms. Preferably only one heteroatom is present which is most preferably O.

Halogen means fluorine, chlorine, bromine or iodine, preferably chlorine.

Homocyclic or heterocyclic ring denote for a saturated or unsaturated 5-6-membered ring such as a pyrrolidine, piperidine, morpholine or pyrroline ring.

The homocyclic and heterocyclic rings can be optionally substituted once or twice by C₁-C₆ alkyl groups, preferably a methyl, ethyl or isopropyl groups as well as by chlorine, bromine or hydroxy, methoxy, benzyloxy, amino, methylamino, dimethylamino or benzylamino groups.

Aryl usually denotes a phenyl residue which is optionally substituted once or several times.

Heteroaryl usually denotes a pyridine, pyrimidine, pyridaszine, pyrrole, thiophene, furan or imidazole ring which is substituted once or several times.

Bicyclic aryl usually denotes an indane or naphthalene residue which is optionally substituted once or several times, preferably a naphthalene residue.

Aryl, bicyclic aryl and hetaryl residues can be optionally substituted once or several times by C₁-C₆ alkyl groups, preferably a methyl, ethyl or isopropyl group as well as by chlorine, bromine, fluorine or hydroxy, alkoxy such as e.g. methoxy, benzyloxy, acetyloxy, carboxy, ethoxycarbonyl, aminocarbonyl, methylaminocarbonyl, dimethylaminocarbonyl, cyano, amino, methylamino, dimethylamino, benzylamino, acetylamino, benzoylamino and amidine groups.

Arylalkyl usually denotes an unsubstituted or once or several-fold substituted benzyl, phenethyl, phenyl-propyl, phenylbutyl or phenylpentyl residue, preferably a benzyl, phenethyl or phenylpentyl residue. C₁-C₆ alkyl residues, preferably a methyl, ethyl or isopropyl group as well as chlorine, bromine, fluorine or hydroxy, methoxy, benzyloxy, acetyloxy, carboxy, ethoxycarbonyl, aminocarbonyl, methylaminocarbonyl, dimethylamino-carbonyl, cyano, amino, methylamino, dimethylamino, benzylamino, acetylamino, benzoylamino and amidino groups come into consideration as substitutes.

Arylheteroalkyl, heteroarylheteroalkyl and heteroarylalkyl denote for the same residues as arylakyl but there is at least one carbon substituted by a heteroatom.

C₁-C₁₀-alkyl and C₁-C₁₀-heteroalkyl, denote for a straight chained branched group with 1 to 10 backbone atoms.,

Amino acid denote for proteinogenic amino acids and non-proteinogenic amino acids. Examples for those aminoacids are: 2-Amino-2-Methylbutanecarboxylic acid, 2-Fluoro-β-Alanine, β-Alanine, 2,3-Diaminosuccinic acid, β-Aminoisobutyric carboxylic acid, Isoserine, 2-Amino-3-Hydroxy-4-Methylpentanecarboxylic acid, 2-Amino-3-Methoxybutanecarboxylic acid, Diaminopropionic acid, 2-Amino-2-Methyl-3-Hydroxypropanecarboxylic acid, 2-Amino-2-Methylbutanedicarboxylic acid, 2-Amino-3-Hydroxy-3-Methylbutanecarboxylic acid, 2,3-Diaminopropionic acid, 2-Amino-2-Methyl-3-Hydroxypropanecarboxylic acid, 2-Amino-2-Methylbutane dicarboxylic acid, 2-Amino-2-Methyl-4-Pentenecarboxylic acid, 2-Amino-3-Methoxypropanecarboxylic acid, 1-Amino-1-Cyclohexanecarboxylic acid, 1-Amino-1-Cyclopentanecarboxylic acid, 1-Aminocyclobutanecarboxylic acid, 1-Aminocyclopropanecarboxylic acid, 2-(2-Furyl)-Glycine, 2-Amino-3-Fluorobutyric acid, 2-Aminoisobutyric acid, 3-Chloro-Alanine, 3-Fluoro-Norleucine, 3-Fluoro-Valine, 3-Fluoroalanine, 3-Methoxy-Valine, α-Cyano-Alanine, α-Methyl-Leucine, β-Chloro-Alanine, β-Cyano-Alanine, β-Hydroxy-Leucine, β-Hydroxyaspartic acid, 3-Hydroxyaspartic acid, 2-Aminobutyric acid, Allylglycine, γ-Methylleucine, Homoserine, Norleucine, Norvaline, tert-Leucine, 2,3-Diaminosuccinic acid, 2-Amino-4-Pentenecarboxylic acid, 2-Aminoheptanecarboxylic acid, 2-Cyclopropyl-2-Methylglycine, 4-Thiaisoleucine, Allothreonine, α-Methylaspartic acid, α-Methylserine, β-Hydroxynorvaline, β-Methylaspartic acid, Homocysteine, O-Methylserine, Penicillamine, Propargylglycine, Vinylglycine, H-4,5-Dehydro-Leu-OH, H-α-Me-Val-OH, H-Propargyl-Gly-OH, H-Allo-Ile-OH, H-Pra-OH, H-Trans-4,5-Dehydro-Lys-OH, 3-Hydroxyaspartic acid, 6-Hydroxynorleucine, Allo-Isoleucine, Allyl Glycine, α-Amino-N-Butyric acid, γ-Methylleucine, α,β-Diaminosuccinic acid, O-Carbamoyl-Serine, S-Methyl-Cysteine, Citrulline, Cyclohexylalanine, α,γ-Diaminobutyric acid, α,β-Diaminopropione acid, Methionine-Sulfoxide, Cα-Methyl-Alanine, N-Methyl-Glycine (Sarkosine), Naphtylalanine, Ornithine, 1,2,3,4-Tetrahydroisochinoline-3-carboxylic acid, Homocysteine, 4-Hydroxy-Proline, 5-Hydroxy-Lysine, Aminobutyric acid, Pantonine, Glucosamine acid, Lanthionine, Aliine, Dopa, Canavanine, Oletopine, β-Lysine, β-Alanine.

The term "inhibition" according to the invention means a substantial reduction of MMP activity in vitro and in vivo. The MMP activity cmi be determined in vitro, for example, in an enzyme assay according to F. Grams (1993) (vide infra). "Substantial inhibition" means an inhibition of at least about 50%, preferably at less than mmolar concentration of the inhibitor (based on MMP activity without an inhibitor). Generally, an inhibition of more than about 80% to 90% is found.

### Preparation of the compounds of the invention.

The compounds of formula (1) can be prepared by acylating an alpha amino acid with an activated derivative of a sulfonic acid e.g. a sulfonyl chloride. Sulfonyl chlorides can be prepared by known processes by reacting a sulfonic acid with e.g. PCl₅ or thionylchloride. The starting sulfonic acids are known or can be prepared using known procedures. The acylation of amino acids can be done in aqueous solvents at a basic pH, in organic solvents in the presence of a tertiary amine as base or under neutral conditions using persilylated aminoacids as starting materials or methyl trimethylsilyl dimethylketeneacetal as a proton trap. The amino acids may be protected at the carbonyl group e.g. as benzyl or methyl, ethyl or trimethylsilylesters. The protecting groups are split off by hydrogenation in the case of a benzyl ester or by alkaline hydrolysis. Trimethylsilylesters are most easily hydrolyzed by water or aqueous solvents. The required alpha amino acids are known or can be prepared using known procedures which are described in Houben-Weyl Vol XI, 304, Houben-Weyl Vol. E5, 504.

### Example 1

### N-(p-Chloro-biphenylsulfonyl)-S-benzyl-cysteine

0.2 g of L-S-benzylcysteine are suspended in 10 ml dichloromethane. 0.31 ml of trimethylsilylchloride are added under exclusion of moisture. The mixture is heated under reflux for 30 minutes, 0.34 ml triethylamine are added and the mixture further refluxed for another 30 minutes. The resulting solution is treated at 0 - 5 °C with 0.11 ml N-methylmorpholine, 10 mg of p-dimethylaminopyridine and 231 mg of p-chlorobiphenylsulfonic acid chloride. The mixture is kept at 0 - 5 °C for 20 minutes an then for 2 hrs. at room temperature and finally poured into sodium bicarbonate solution. The solution is extracted with dichloromethane. Then, the aqueous layer is acidified with HCl and the product extracted with dichloromethane. The organic solvent is evaporated and the residue is chromatographed on silica gel using dichloromethane/ 2% acetic acid as eluent to yield 0.16 g (35%) of the title compound.
DC: (SiO₂ dichloromethane/acetone/acetic acid 9:1:0.3) Rf=0.46
MS: (APCI) M-H = 459.9

Using the procedure of example 1 the amino acid listed in the table is reacted with p-chloro-biphenylsulfonyl chloride to give the compound in the table.

### Example 2

| **Amino acid** | **Product** | **MS (APCI)** | **Rf (SiO**_{**2**}**)** |
|---|---|---|---|
| L-Aspartic acid β-benzylester | L-N-(p-Chlorobiphenylsulfonyl)-aspartic acid-β-benzylester | 472.0 (M-H) | 0.4 CH₂Cl₂/acetone/acetic acid (9:1:0.3) |

The following selection provide an illustration of embodiments of the invention and should not be construed to limit the scope of the invention:

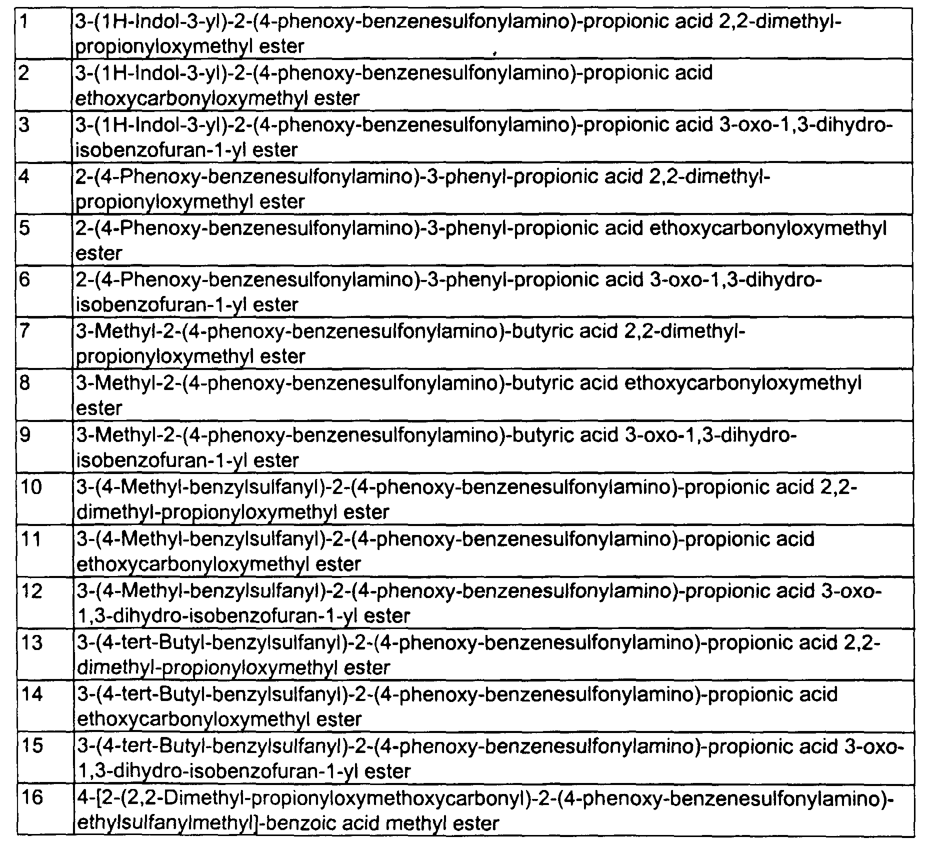

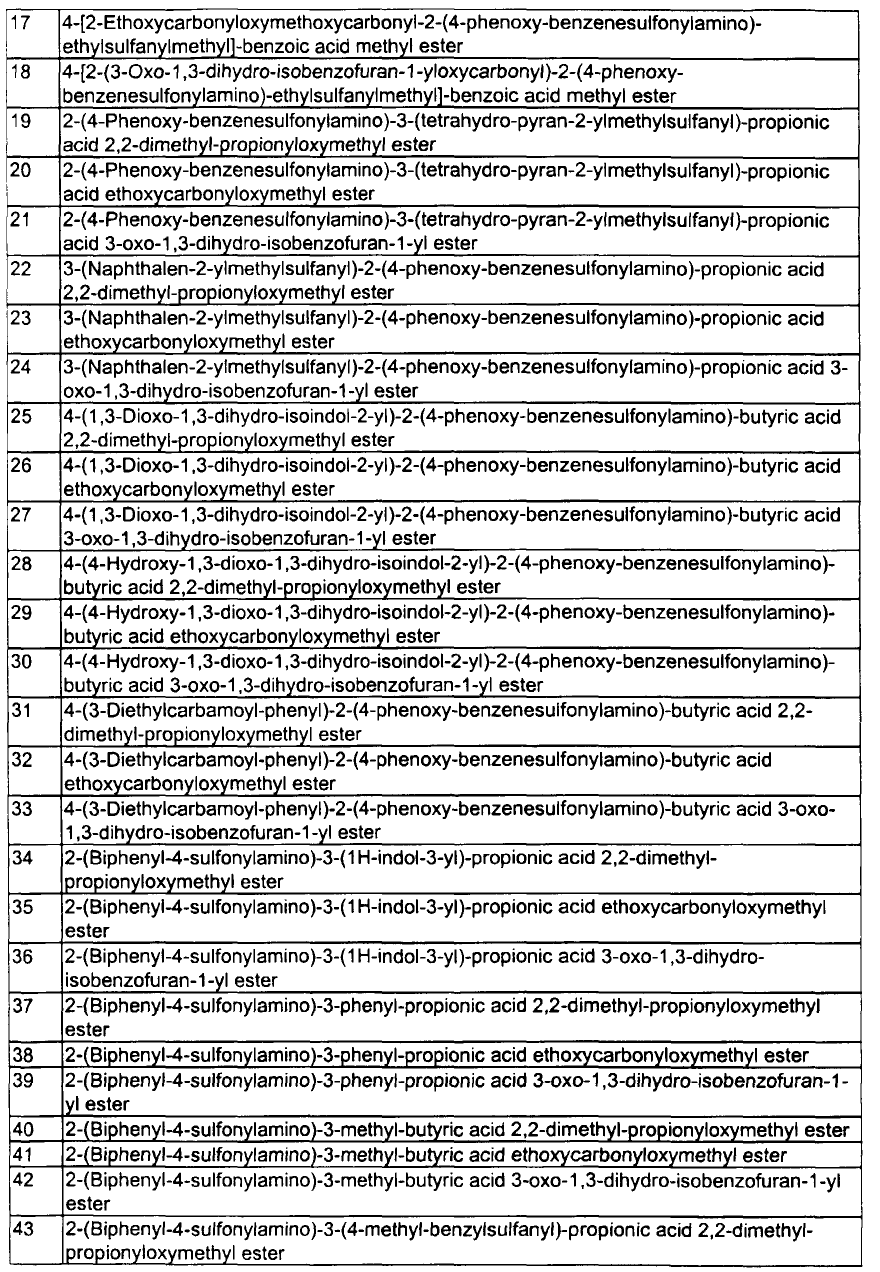

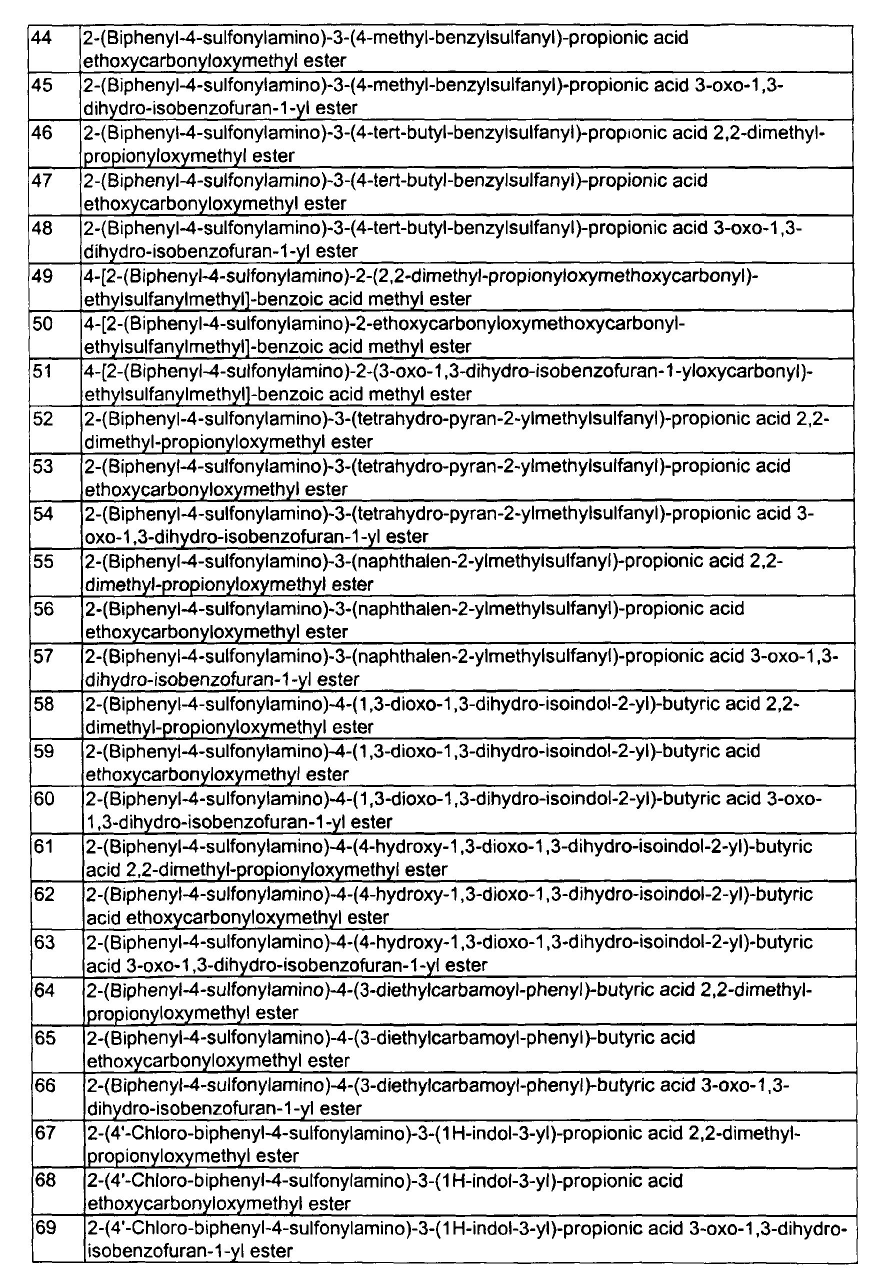

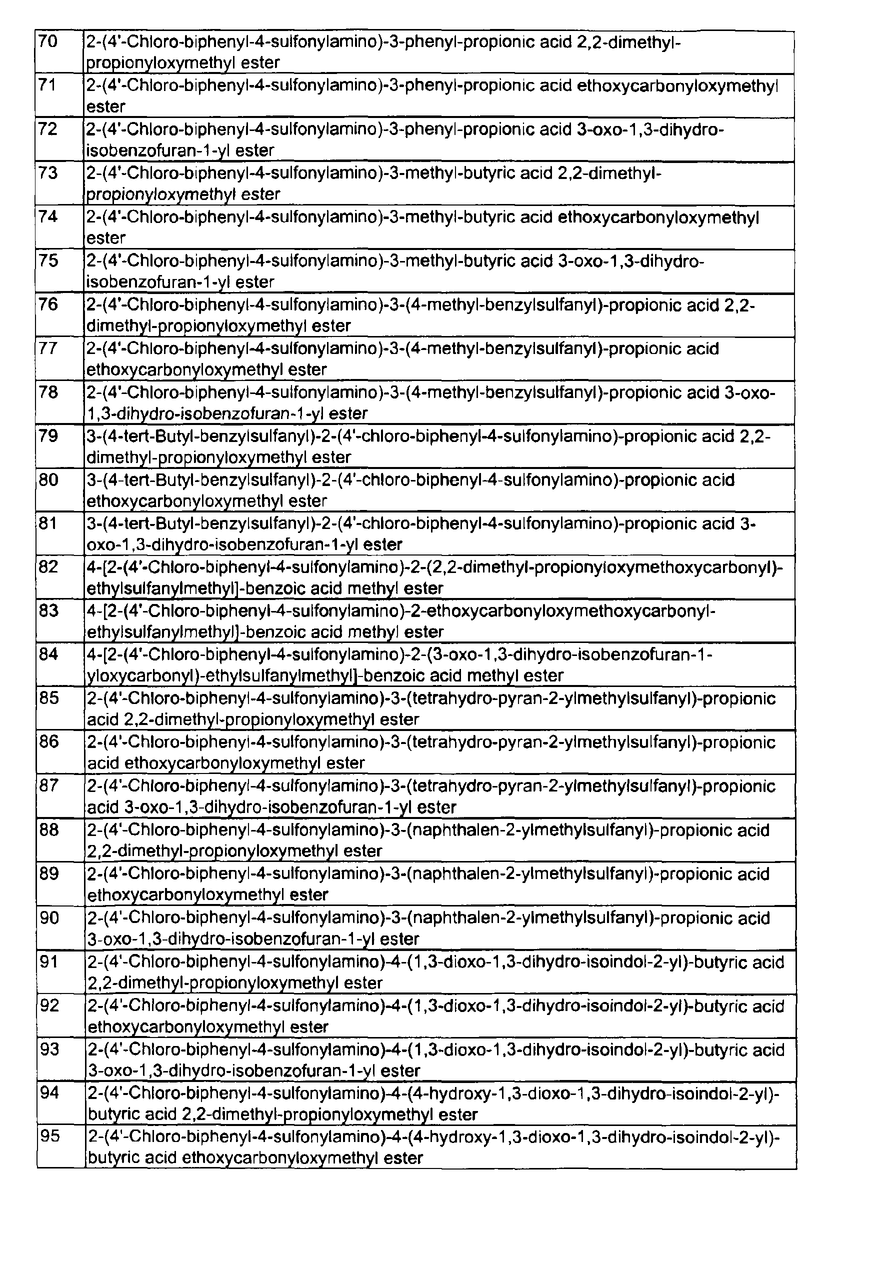

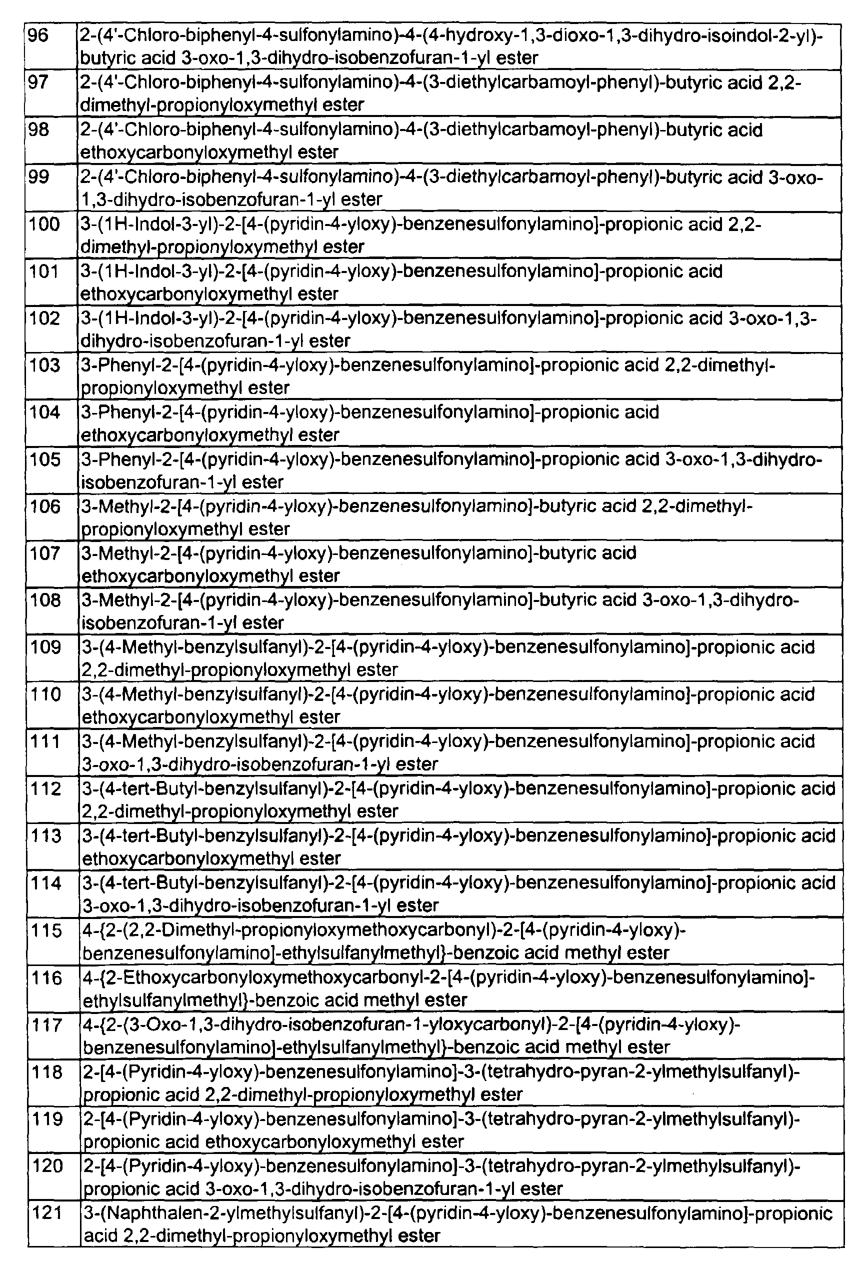

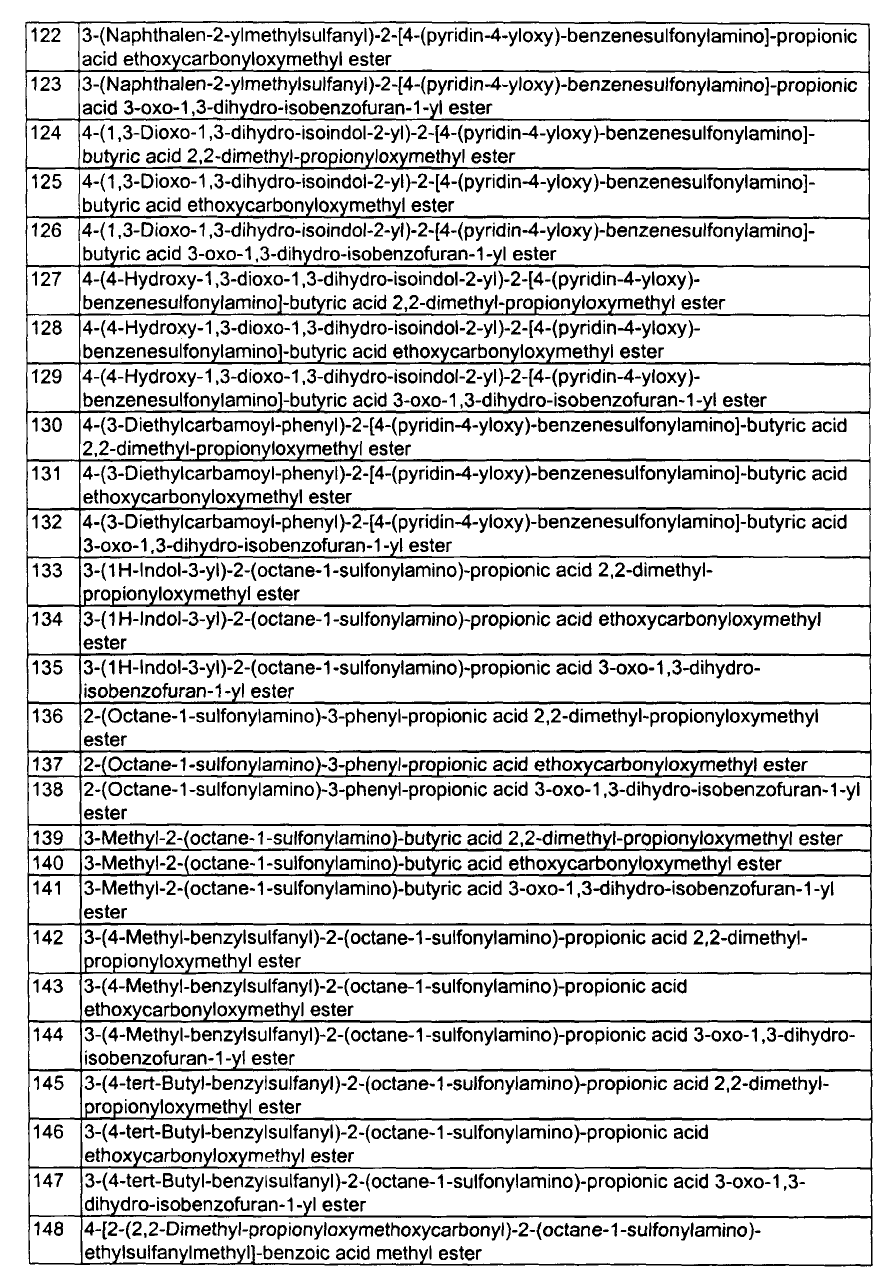

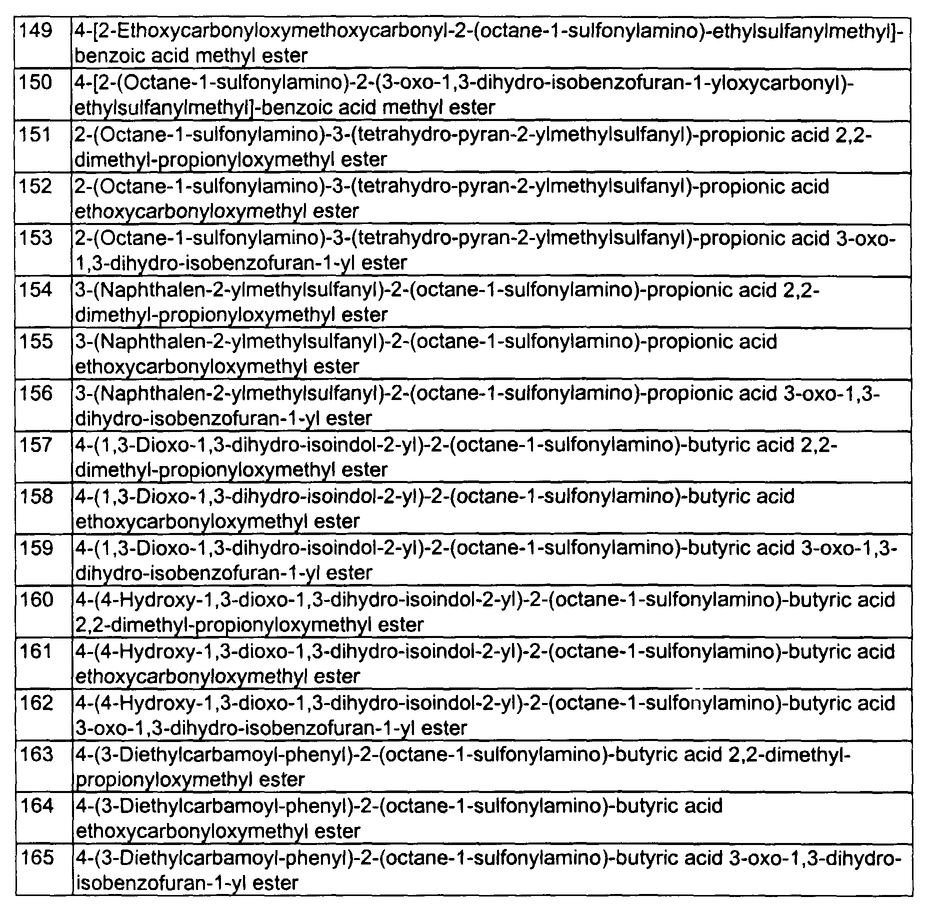

Compounds of the general formula I can contain one or several chiral centres and can then be present in a racemic or in an optically active form. The racemates can be separated according to known methods into the enantiomers. Preferably diastereomeric salts which can be separated by crystallization are formed from the racemic mixtures by reaction with an optically active acid such as e.g. D- or L-tartaric acid, mandelic acid, malic acid, lactic acid or camphorsulfonic acid or with an optically active amine such as e.g. D- or L-α-phenyl-ethylamine, ephedrine, quinidine or cinchonidine.

Alkaline salts, earth alkaline salts like Ca or Mg salts, ammonium salts, acetates or hydrochlorides are mainly used as pharmacologically acceptable salts which are produced in the usual manner e.g. by tritrating the compounds with inorganic or organic bases or inorganic acids such as e.g. sodium hydroxide, potassium hydroxide, aqueous ammonia, C₁-C₄-alkyl-amines such as e.g. triethylamine or hydrochloric acid. The salts are usually purified by reprecipitation from water/acetone.

The new substances of formula I and salts thereof according to the invention can be administered enterally or parenterally in a liquid or solid form. In this connection all the usual forms of administration come into consideration such as for example tablets, capsules, coated tablets, syrups, solutions, suspension etc. Water which contains additives such as stabilizers, solubilizers and buffers that are usual in injection solutions is preferably used as the injection medium.

Such additives are e.g. tartrate and citrate buffer, ethanol, complexing agents (such a ethylenediaminetetra-acetic acid and non-toxic salts thereof), high-molecular polymers (such as liquid polyethylene oxide) to regulate viscosity. Liquid carrier substances for injection solutions have to be sterile and are preferably dispensed into ampoules. Solid carrier substances are e.g. starch, lactose, mannitol, methylcellulose, talcum, highly dispersed silicic acids, higher molecular fatty acids (such as stearic acid), gelatins, agar-agar, calcium phosphate, magnesium stearate, animal and vegetable fats, solid high-molecular polymers (such as polyethylene glycols); suitable preparations for oral application can optionally also contain flavourings and sweeteners.

The dosage can depend on various factors such as manner of administration, species, age and/or individual state of health. The doses to be administered daily are about 1 - 10000 mg, preferably 5-2000 mg/human, more preferably 100-500 mg/human and can be taken singly or distributed over several administrations.

Prodrugs of the compounds of the invention are such which are converted in vivo to the pharmacological active compound. The most common prodrugs are carboxylic acid esters, e.g. acetats, ethyl esters etc.

### Example 3

In order to determine the inhibition of MMPs, for example MMP-8, the catalytic domain (isolation and purification see for example Schnierer, S., Kleine, T., Gote, T., Hillemann, A., Knäuper, V., Tschesche, H.,Biochem. Biophys. Res. Commun. (1993) 191, 319-326) is incubated with inhibitors having various concentrations. Subsequently, the initial reaction rate in the conversion of a standard substrate is measured in a manner analogous to Grams F. et al., FEBS 335 (1993) 76-80). Alternatively the percent inhibition will be determined at a distant inhibitor concentration.

The assays for other MMPs are performed in a analogous manner.
Assay buffer: 50 mM Tris/HCI pH 7.6 (Tris= Tris-(hydroxymethyl)-aminomethan) 100 mM NaCl/10 mM CaCl₂-/5% MeOH (if necessary)
Enzyme: 8 nM catalytic domain (Met80-Gly242) of human neutrophil collagenase
Substrate: 10 microM DNP-Pro-Leu-Gly-Leu-Trp-Ala-D-Arg-NH2
Total assay volume: 1 ml

Table 1 shows the inhibition in % at a compound concentration of 100 ng/ml or IC50 values in brackets.

**Table 1**

| **Example** | **MMP-8 Inhib. [%] IC50 [nM]** | **MMP-9 Inhib. [%]** |
|---|---|---|
| 1 | 21.6 nM | |
| 2 | 48.7 nM | 38%/0.21 µM |

## Claims

1. Use of a compound of formula I 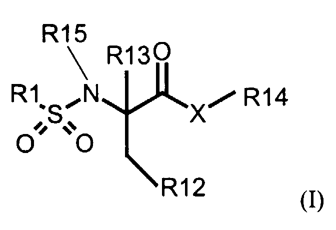 wherein R₁ is one of the following residues: 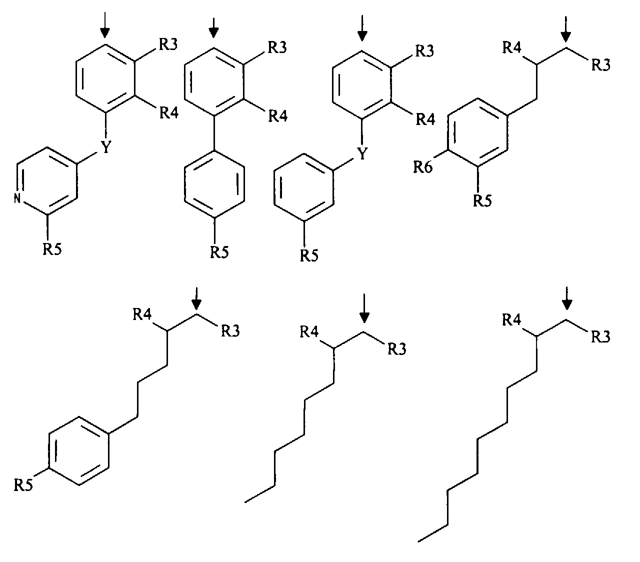 whereby
R3 and R4 independently have the one of the meanings H, C₁-C₁₀-alkyl, aryl, aryl-C₁-C₆-alkyl, C₁-C₁₀-heteroalkyl, heteroaryl, heteroaryl-C₁-C₆-alkyl, heteroaryl-C₁-C₆-heteroalkyl, aryl-C₁-C₆-heteroalkyl, and
R3 and R4 are optionally in the case of a phenyl substitution in addition independently of each other NR7R8
R7 and R8 is independently of each other hydrogen or lower alkyl
R3 and R4 are optionally in the case of an alkyl substitution in addition independently of each other COR10
R10 is hydroxy or C1-C10 heteroalkyl
R5 and R6 independently have the meanings H, OH, halogen, lower alkyl, lower alkenyl, or lower heteroalkyl, preferable at least one is hydrogen,
Y has the meanings O, S, NH, (CH₂)ₙ, n is 1, 2 or 3,
R12 is a residue of 5 to 21 backbone atoms, said residue being saturated or unsaturated, linear or branched, and contains optionally homocyclic or heterocyclic structures, or denotes an aralkyl or is represented by -(CH2)n-W-R12' with n = 0 - 4 and W = S, O, NH and R12' is the remaining moiety of R12, but not H or methyl.
R13 represents H, lower alkyl, lower alkenyl, phenyl or benzyl.
R14 denotes H, alkyl, alkenyl, aryl, aralkyl or can form together with X, if X is N, a C-terminal protected or unprotected amino acid or denotes the formula -CH(R15')-O-CO-R16 wherein R15' stands for H, alkyl, alkenyl, aralkyl or aryl and R16 has the meaning of optionally substituted alkyl, alkoxy, aralkyl, aralkyloxy or R15' and R16 together may form a five or sixmembered ring, which may be optionally substituted or condensed with further carbocyclic or heterocyclic rings,
R15 is H, lower alkyl, aryl, heteroaryl, heteroaryl-lower-alkyl and
X denotes O or N,
with the proviso, that R14 is not H, if X stands for O and the configuration of the C-atom with the substituents R13, N, CO and CH₂R12 is derived from a D-amino acid with R13 is H; and with the provisio that R14 cannot be H, C1-C6-alkyl, benzyl or t.-butyl if X is O and R1 is a biphenyl residue; and with the provisio that R14 can be H if X denotes O, R1 is biphenyl and if R12 is represented by the group -(CH2)n-W-R12' wherein W denotes S and R12' is the remaining moiety of R12 but not H or methyl,
as well as their racemates, diastereomers, pharmacologically acceptable salts or prodrugs thereof, to produce pharmaceutical agents for the treatment of diseases where MMP activity is involved.

2. Use as claimed in claim 1 wherein R1 shows a logP-value between 1.5 and 5.5 if calculated as H-R₁ with the proviso that at least the beginning three backbone atoms of the chain are carbon atoms, with the proviso that there are maximal 3 heteroatoms in the chain and with the proviso that there are at least 2 carbon atoms between each pair of heteroatoms in the chain.

3. Use as claimed in one of the claims 1 or 2 wherein the length of the relaxed residue R1 is in the range from 6 to 10 Ångstroms, the maximal width of R1 is 3.5 Å (all dimensions are calculated without H-atoms) and the volume of the connolly surface of R1 is between 150 and 250 Å³.

4. New compounds of formula I 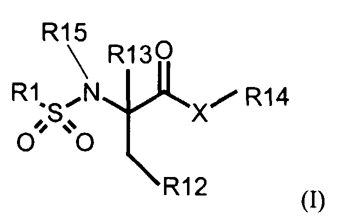 wherein
R₁ is represented by the following residues: 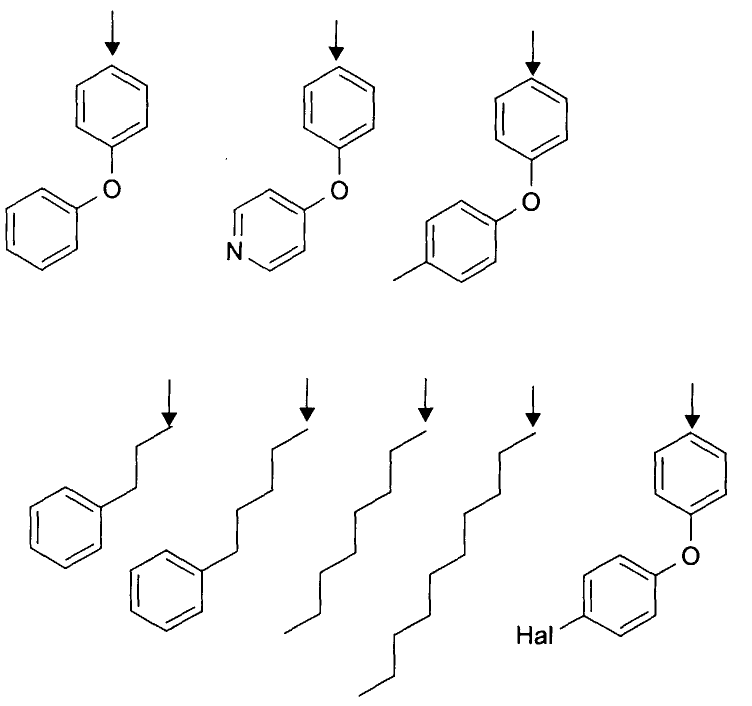
R12 is a residue of 5 to 21 backbone atoms, said residue being saturated or unsaturated, linear or branched, and contains optionally homocyclic or heterocyclic structures, represented by -(CH2)n-W-R12' with n = 0 - 4 and W = S, and R12' is the remaining moiety of R12, but is not H or methyl,
R13 represents H, lower alkyl, lower alkenyl, phenyl or benzyl,
R14 denotes H, alkyl, alkenyl, aryl, aralkyl or can form together with X, if X is N, a C-terminal protected or unprotected aminoacid or denotes the formula -CH(R15')-O-CO-R16 wherein R15' stands for H, alkyl, alkenyl, aralkyl or aryl and R16 has the meaning of optionally substituted alkyl, alkoxy, aralkyl, aralkyloxy or R15' and R16 together may form a five or sixmembered ring, which may be optionally substituted or condensed with further carbocyclic or heterocyclic rings,
R15 is H, lower alkyl, aryl, heteroaryl, heteroaryl-lower-alkyl and
X denotes O or N,
as well as their racemates, diastereomers, pharmacologically acceptable salts or prodrugs thereof.

5. Pharmaceutical composition containing at least one compound of the general formula I as claimed in claim 4 in addition to the usual pharmaceutical auxiliary substances and carriers.

6. Use of a compound of formula I as claimed in claim 4 for the production of pharmaceutical agents for the treatment of osteo- and rheumatoid arthritis, periodontal diseases, diseases, where the presence of bacteria results in the release of MMPs, corneal ulceration, metastasis, angiogenesis, tumour progression, multiple sclerosis, sun-induced skin-aging, emphysema, and cardiovascular diseases.
